# EUROPEAN PATENT SPECIFICATION

(11) **EP 3 908 334 B1**
(45) Date of publication and mention of the grant of the patent: **11.12.2024**
(21) Application number: 20738556.8
(22) Date of filing: 10.01.2020
(51) Int. Cl.: A61L 2/232, A01N 33/12, A01N 25/34, A61L 2/18, A01N 43/40, A01N 47/44, A01N 55/00, D21H 27/30, D21H 27/32, B32B 29/00, A61K 8/02, A61K 9/70, A61Q 19/00, A61P 31/04, A61P 31/14, A61K 31/14, A61K 31/355, A61K 31/155, A61K 36/886

(54) **IMPROVED METHOD AND COMPOSITIONS FOR SURFACE TREATMENT**
VERBESSERTES VERFAHREN UND ZUSAMMENSETZUNGEN ZUR OBERFLÄCHENBEHANDLUNG
PROCÉDÉ ET COMPOSITIONS AMÉLIORÉS POUR TRAITEMENT DE SURFACE

(30) Priority: 11.01.2019 AU 2019900082
(43) Date of publication of application: 17.11.2021
(73) Proprietor: Kritzler, Steven, Cronulla, NSW 2230 (AU)
(72) Inventor: Kritzler, Steven, Cronulla, NSW 2230 (AU)
(74) Representative: Dehns
(86) International application number: PCT/AU2020/050014
(87) International publication number: WO 2020/142813

(56) References cited:
- WO-A1-2013/036996
- WO-A1-2015/189568
- WO-A1-2015/189570
- WO-A2-2007/070649
- JP-A- 2018 015 413
- US-A- 5 421 898
- US-A- 5 707 736
- US-A1- 2004 009 141
- US-B2- 7 741 263
- HINCHLIFFE, D. J. ET AL.: "An optimized co-formulation minimized quaternary ammonium compounds adsorption onto raw cotton disposable disinfecting wipes and maintained efficacy against methicillin-resistant Staphylococcus aureus, vancomycin- resistant Enterococcus faecalis, and Pseudomonas aeruginosa", TEXTILE RESEARCH JOURNAL, vol. 88, no. 20, 2018, pages 2329 - 2338, XP055724487

## Description

### Field of the Invention

This invention relates to improved methods, compositions and devices for delivering active agents from cellulosic substrates to animate and inanimate surfaces. Preferred embodiments of the invention may be used to impart a specific change to one or more properties of the surface so treated, for example, reduced microbial colonisation on surfaces treated by the substrates of the invention, or improved emolliency or skin feel or other desired properties.

### Background

Any discussion of the prior art throughout the specification should in no way be considered as an admission that such prior art is widely known or forms part of common general knowledge in the field.

It is usual for compositions used to treat surfaces to be supplied in jars, bottles, cans, or other containers which are suitable for storage, transport and commercial use. When used, the compositions are wiped over the surface using woven cloths, non-woven fabrics or paper or the like to transfer the composition from the container to the surface and then to spread it in a film on the surface.

While some compositions are supplied ready for use in wet form where they are preimpregnated in non-woven fabrics, compositions containing cationic components have not been supplied in the form of a treated paper or other cellulosic substrate. This is because it is well known that cellulose and its various derivatives are significantly anionic in character and therefore would be expected to very effectively deactivate cationic molecules.

Hinchcliffe et al (Textile Res Journal, 2018, 88(20) 2329-2338) solve the problem of deactivation of cationic species by cellulosic substrates by the use of potassium citrate. However, having trialled the use of potassium citrate incorporated into our various aqueous cationic cellulosic substrate treatments, virtually no beneficial effects were found probably because the cationic strength of the active molecules in the treatments provided strong attraction to the anionic sites on the substrate.

The inventor's earlier patent application, PCT/AU2006/000130, described a method and composition for the prevention of microbial colony growth on a hard surface which involved coating a surface, for example a hospital surface or a food preparation surface, with a cationic solution or emulsion of a specified composition and then wiping it to spread the liquid before allowing it to dry. Wiping the composition onto the surface cleans the surface, disinfects the surface, and leaves a residual coating which renders the surface bacteriostatic for extended periods of time, which can typically be measured in weeks. The specified compositions in that case consisted essentially of a biostatic complex formed between a polyvinyl alcohol (hereinafter also referred to as "PVOH") and a C12-C18 straight chain alkyl benzalkonium quaternary compound. Those compositions contained from 1.2% to 8% w/w of PVOH and up to 8% w/w of the quaternary ammonium compound. Although such compositions provide major benefits and have met with commercial success, they were understood to suffer from the disadvantage that, because of the highly cationic nature of the composition, it could not be sold impregnated onto a cellulosic substrate, but rather need to be supplied in a container. The user was required to locate an applicator such as a nonwoven or fabric or paper wipe with which to wipe the surface immediately after application of the composition.

WO 2015/189568 (FANTEX LTD) relates to a liquid antimicrobial composition comprising: (a) water, (b) a water-soluble polymer, and (c) at least one water-soluble antimicrobial; as well as to an item of clothing, a curtain, a blind, an item of bedding, wallpaper or laundry product comprising the water-soluble polymer and the at least one water- soluble antimicrobial. WO 2015/189568 also relates to a method of preparing the liquid antimicrobial composition.

WO 2007/070649 (3M INNOVATIVE PROPERTIES CO) describes an antimicrobial coating system, a film-forming composition, and an antimicrobial film.

It is an object of the present invention to overcome or ameliorate one of the abovementioned disadvantages, or at least provide a commercial alternative.

### Brief Statement of invention

According to a first aspect the present invention provides an applicator as defined in claim 1.

Preferably, the applicator in the form of a sheet wipe. The sheet wipe comprises multiple layers (for instance, two, three, four, five or more layers, e.g. two or three layers) of cellulosic substrate.

According to one aspect the invention provides an applicator for providing an active composition to a surface, the applicator comprising at least two layers of cellulosic substrate having anionic sites;
said layers being adhered to each other by an adhesive composition, the adhesive composition comprising a cationic component in an amount sufficient in use to deactivate the anionic sites in the cellulosic substrate and to provide a desired effect which is selected from one or more of a biocidal effect, enhanced skin feel, enhanced skin health and fabric softening;
wherein the adhesive composition also comprises a water-soluble or water dispersible adhesive polymer;
wherein the water-soluble or water-dispersible adhesive polymer is selected from one or more of PVOH, polyvinyl pyrrolidone or copolymers of polyvinyl pyrrolidone with vinyl acetate, non-ionic polyvinyl acetate emulsions or vinyl ether/maleic anhydride copolymer; and
wherein the cationic component is a cationic biocide, or a skin compatible cationic selected from benzalkonium chloride, benzethonium chloride, alkylammonium chloride, chlorhexidine gluconate, octenidine hydrochloride, tallow quaternary compounds or silicone-based quaternaries.

The adhesive composition comprises a water-soluble or water-dispersible adhesive polymer. The water-soluble or water-dispersible adhesive polymer is selected from one or more of PVOH, polyvinyl pyrrolidone or copolymers of polyvinyl pyrrolidone with vinyl acetate, non-ionic polyvinyl acetate emulsions or vinyl ether/maleic anhydride copolymer.

The impregnation or coating of the present invention preferably takes place without the need for thermal modification, i.e. there is no thermoplastic adhesion between the substrate and the cationic compound.

In an embodiment, the water-soluble or water-dispersible adhesive polymer is polyvinyl alcohol.

The applicator may be wet or dry. In some embodiments, it is preferable if the applicator is substantially dry.

The cationic component is selected from the group consisting of a cationic biocide which is preferably a quaternary ammonium compound, or a skin compatible cationic selected from benzalkonium chloride, benzethonium chloride, alkylammonium chloride, chlorhexidine gluconate, octenidine hydrochloride, tallow quaternary compounds or silicone based quaternaries.

In one embodiment, each one of said at least two layers are the same. In another embodiment, each one of said at least two layers are different. In some embodiments, the applicator comprises multiple layers of cellulosic substrate adhered to each other by an adhesive composition.

The cellulosic substrate may be selected from the group consisting of paper, paper laminates, cardboard, plant fabrics or the like. The plant fabric may be a wood pulp, cotton material, bamboo material, hemp material or the like. The cellulosic substrate may be woven or non-woven.

Also described is a method of preparing an applicator according to preceding aspects comprising:
selecting a cellulosic substrate and a cationic component,
determining a base loading of the cationic component on the cellulosic substrate at which and anionic sites on the cellulosic substrate are inactive, and
selecting an amount of cationic component for application to the cellulosic substrate such that cationic component will exhibit an effect in use.

The amount of cationic component comprises the base loading and an amount sufficient to provide a desired or predetermined effect.

The cationic component may be selected from the group consisting of a quaternary ammonium compound, or a skin compatible cationic selected from benzalkonium chloride, benzethonium chloride, alkylammonium chloride, chlorhexidine gluconate, octenidine hydrochloride, tallow quaternary compounds or silicone based quaternaries.

The cellulosic substrate may be selected from the group consisting of paper, paper laminates, cardboard, plant fabrics or the like. The plant fabric may be a wood pulp, cotton material, bamboo material, hemp material or the like. The cellulosic substrate may be woven or non-woven.

According to another aspect the invention provides a method of making an applicator comprising:
- contacting the surface of a first ply of cellulosic material with an adhesive composition comprising a cationic component and
- applying to the adhesive composition a further ply of cellulosic material, and
wherein the cationic component is present in an amount sufficient in use to deactivate the anionic sites in the cellulosic substrate and to provide a desired effect which is selected from one or more of a biocidal effect, enhanced skin feel, enhanced skin health or fabric softening.

In the case of a biocidal effect the biocidal efficacy of the treatment is measured using the same standardised methodology as has for decades been used with wet wipes. In the case of wet wipes the liquid is squeezed from the wipe and the biocidal efficacy of this liquid then tested against microorganisms according to standard methods.

In the case of the dry wipes as described herein, where the dry wipe is composed of 2 paper plys each of 20gms/sq.m. The first ply by applying via transfer roller a solution of benzalkonium chloride and polyvinyl alcohol to the surface of this first ply which is then brought into contact and squeezed between rollers to contact the second ply. The quantity of treatment applied to the surface is insufficient to saturate the substrate even after squeezing between rollers so that the now composite structure can be immediately rolled up without the adjacent layers of the 2 ply material bonding to each other.

When this treated substrate has been formulated and manufactured so as to satisfy the requirements of hospital grade hard surface disinfection the antimicrobial efficacy is assessed as follows:
For each 20 × 23 sq.cm section of treated substrate 4 mls of water is added. Soon after this addition the wetted paper is squeezed and the exuded solution collected. This solution is then subjected to standardised microbiocidal efficacy testing as described in the4 Australian Health Therapeutic Goods Administration Option A test. For compliance to this test the solution must achieve an over 5 log reduction in a Staph aureus suspension and also an over 5 log reduction in an E coli suspension.

In the case of a substrate intended for skin antisepsis similar methodology is used whereby a predetermined area of treated substrate is wetted with water, subsequently squeezed and the liquid collected and its antimicrobial efficacy tested by standardised test methods.

The Australian Health Department's Therapeutic Goods Administration has approved this test methodology for the assessment of efficacy of active, dry substrates which are to be activated by water contact.

Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

Preferably, subsequent layers of adhesive and ply are applied to make a multi-ply applicator, and wherein the cationic component is present in an amount sufficient in use to deactivate the anionic sites in the cellulosic substrate and to provide a desired effect.

The adhesive composition comprises a water-soluble or water-dispersible adhesive polymer. It is selected from one or more of PVOH, polyvinyl pyrrolidone or copolymers of polyvinyl pyrrolidone with vinyl acetate, non-ionic polyvinyl acetate emulsions or vinyl ether/maleic anhydride copolymer.

The invention also provides a method of disinfecting an inanimate surface comprising moistening the surface and then wiping it with an applicator according to any one of the preceding aspects.

The invention also provides a method of disinfecting an inanimate surface comprising moistening an applicator according to any one of the preceding aspects then wiping the surface with the moistened paper or fabric whereby to apply a bioactive cationic species to the surface.

The invention also provides a method of disinfecting skin comprising moistening the surface and then wiping it with an applicator any one of the preceding aspects.

The invention also provides a method of disinfecting skin surface, the method comprising moistening an applicator according to any one of the preceding aspects and then wiping the surface with the moistened paper or fabric whereby to apply the a bioactive cationic species to the skin.

The invention also includes treating cellulosic substrates such as cardboard with the above described cationic antimicrobial substances to render long term resistance to microbial colonisation such as fungal colonisation.

As herein used the term "cellulosic" includes cellulose and cellulose derivatives, including chemically reacted cellulosics such as for example cellulose acetate, as well as impure natural forms such as cotton, and wood and may be in the form of paper, paper laminates, cardboard, fabrics such as woven cotton fabrics or the like. Wood pulp or cotton materials are preferred, particularly in the case where the applicators are provided in a wet form. The cellulosic material may be in woven or non-woven form.

For preference the cellulosic fibres are formed into in paper sheets or laminates or are in the form of in cotton fabrics. The applicator is, for preference, a sheet wipe. Most preferably, the final form of the article is presented to the consumer as a dry article, so the above processes can include a drying step, such as air drying, oven drying, contact drying etc.

It should be noted that cellulosic materials which feel dry actually contain a substantial portion of moisture, so a dry feeling is qualitative rather than quantitative.

The cellulosic substrates may also comprise two or more cellulosic materials, for example, the cellulosic substrate may comprise a cotton scrim and a treated paper layer, or a two cotton scrim layers with a treated paper layer interposed between the scrims.

The cationic species may be a quaternary ammonium compound for instance a benzalkonium chloride, alkylammonium chloride, benzethonium chloride, or it may be another cationic biocide such as a biguanide, for example chlorhexidine gluconate or octenidine hydrochloride. Skin compatible cationic species are particularly preferred. Alternatively, the active cationic molecule may be a tallow quaternary compound, which can be used to achieve fabric softening. In a further example the active quaternary molecule can be an organic or silicone-based molecule to achieve emolliency or other feature of feel to the skin.

The present applicators are preferably free from potassium citrate.

The present inventor has now found, surprisingly, that compositions such as described in PCT/AU2006/000130 can be stored for long periods substantially dry in sheets of nonwoven or woven cellulosic fabrics, for example sheets of paper or paper laminates, and in that form can be stored and transported and then can later be applied to surfaces simply by wetting the surface to be treated or moistening the sheet and wiping the surface to impart both disinfection and biostatic properties to the surface. This significantly reduces the costs of packaging, the costs of supply of the composition, and greatly increases the convenience of use and application to surfaces whilst maintaining a high degree of efficacy.

Surprisingly, the cationic compositions in conjunction with any suitable water-soluble or dispersible adhesive polymer such PVOH, polyvinyl pyrrolidone, it copolymers with vinyl acetate, polyvinyl acetate emulsions which are not anionic, vinyl ether/maleic anhydride copolymer solutions or other such suitable polymer solutions which are adhesive with respect to the cellulosic substrates which are prepared as solutions or emulsions can also act as an adhesive between layers of cellulosic fibres and can be utilized to bond together anionic sheets of paper or other cellulosic substrate to form laminates in the present invention, the laminates being rolled up and or cut into sheets. This synergistically reduces the cost of the multi-ply paper used for application of the cationic composition, since the cost of adhering the ply and providing the cationic treatment are combined into a single step. Using an adhesive composition containing the cationic active as the multi-ply adhesive reduces the overall cost in comparison with applying a cationic composition to a conventionally bonded multi ply paper or fabric

Those skilled in the art would be well aware that paper and other cellulosic materials contain a large number of anionic sites and would not therefore be used for the reversible storage of intrinsically cationic materials. What has not been appreciated previously is that the cellulosic substrates, whilst anionic, have a quite finite, and in many cases modest, anionic character such that if a composition which is substantially cationic is applied to the cellulosic substrate in sufficient concentration then only a portion of the cationic composition is deactivated (a base loading) with the remainder of the cationic material retaining its cationic nature and being available to achieve its intended effect.

Whilst compositions described in PCT/AU2006/000130 were applicable to hard surfaces they were not applicable to skin because some benzalkonium chloride components can cause rashes on skin. Many of the compositions contemplated by the present invention are skin compatible and bring some additional unexpected advantages.

The cationic compound may be applied directly to the cellulosic substrate, for example by transfer rollers, dipping, painting or spraying. Alternatively, in one preferred embodiment of the present invention, two or more layers of cellulosic paper or cellulosic fabric (which may be independently a woven or a non-woven fabric formed from wood pulp, cotton or other substantially cellulosic fibres) are bonded by an intervening adhesive layer which contains PVOH and the cationic material

In one particular embodiment, the present invention provides a substantially dry, 2-ply paper or other cellulosic structure made by coating the contact surface of one of the plys with the formulation containing the active cationic component and PVOH (for example, that shown in Example Formulation 1) and immediately thereafter bringing the coated ply it into contact with the surface of the second ply. The 2-ply structure thus formed can be dried if required and variously cut or formed into a roll. At least one ply in the thus formed applicator is composed of cellulose in its various forms such as paper, cotton or bamboo or a cellulose derivative such as cellulose acetate. The substrates may be in the form of a composite cellulose containing structure such as cardboard or fabrics or a fabric composite.

The paper or fabric may be formed into paper towels which when dry can be used to wipe wet hands immediately after washing. As the towels become wet some of the cationic active, present in an amount in excess of the base loading, immediately dissolves, transferring the cationic active to the skin. In the example of the biocidal active, the biocide can the kill the majority of bacteria left on the skin. The method kills 99.9% of the bacteria and leaves the hands with residual antimicrobial protection for at least up to an hour.

Those skilled in the art will appreciate that hand disinfection is an essential part of avoiding cross infection in hospitals and the like. The frequent application of disinfection compositions to hands causes inconvenience to hospital staff and frequently results in skin irritation. It can also leave the hands feeling greasy which can interfere with secure holding of instruments and the like. Tests conducted in secrecy have shown that personnel prefer to wipe their hands with a dry paper towel such as provided by this invention (when a similar level of disinfection can be obtained and with residual protection) rather than to rub their hands with conventional hand disinfectants.

As mentioned above, compositions described above have not heretofore been applied to cellulosic substrates because it is well known that cellulose and its various derivatives are significantly anionic in character and therefore very effectively deactivate cationic molecules such as those described above.

In further aspect, the invention provides a method of disinfecting skin comprising moistening the skin surface, for example by washing, and then contacting the moistened skin with a paper or fabric wipe containing a skin compatible active cationic molecule in accordance with the present invention. Alternatively, the invention provides a method of disinfecting skin comprising moistening a paper or fabric wipe containing a skin compatible active cationic molecule according to present invention and then contacting the skin surface with the moistened paper or fabric wipe. In both of the above aspects, the skin compatible biocide or other cationic functional molecule is applied to the skin.

### Preferred embodiments of the invention

By way of example only various embodiments of the invention are herein described.

### Example 1. Adhesive composition

This example relates to a composition for use in bonding together two or more thicknesses or layers of cellulose paper (hereinafter referred to as "plys"). As explained above, the present invention can be either 2 ply or more plys.

A solution composed of water into which is dissolved polyvinyl alcohol and a cationic quaternary biocide such as a benzalkonium or alkylonium chloride is prepared. This solution is then applied at a predetermined rate by transfer roller, by spraying, by doctor blade or any other appropriate means to a paper surface which can be stand alone or the first ply of a 2-ply paper structure. The second ply can then be brought into direct contact with the surface of the first ply onto which the cationic composition has been applied. The structure is then compressed between one or more sets of rollers with the cationic solution acting as an adhesive which is quickly absorbed into the paper structure which is substantially dry on the outer surfaces and can therefore be rolled onto a mandrel at high speed without the various layers of the roll sticking one to another.

The rate of addition and concentration of the cationic solution is such that the cationic molecules coated onto the cellulosic substrate result in excess residual cationic material remaining active after a section of the treated 2 ply paper is thoroughly wetted with water.

The treated paper as described above can be wetted with water or used to wipe a substrate which is wet with water. The combination with the quaternary biocide in the paper quickly dissolves and disinfects the substrate which has been wiped. The efficiency and speed of disinfection is controlled by the concentration of the excess quaternary biocide which has been applied to the paper and remains active after the paper has deactivated a portion of the cationic biocide.

Since various different grades and thicknesses of paper or other cellulosic substrates such as cellulose acetate have different degrees of anionic characteristics, the concentration and dosage rate of the cationic solution used to treat the cellulosic structure must be varied to suit the particular circumstance.

Compositions described in PCT/AU2006/000130 contained from 1.2% to 10% w/w of PVOH and up to 11% w/w of quaternary ammonium compound. For celluloses tested to date, the present invention generally has in excess of 8% w/w of quaternary ammonium compound.

Examples of cellulosic substrate formulations suitable for use in example 1 follow.

### Formulation 1:

Formulation 1 is a benzalkonium chloride composition, applied at a rate of 2.20 - 2.95 gm/m² active to achieve compliance with the Australian Therapeutic Goods Administration TGO 54, Option B test under dirty conditions. Higher rates of dosage will also achieve compliance.

| Chemical name (active/ grade) | Contents (w/w)% | Maker / Supplier |
|---|---|---|
| Polyvinyl alcohol | 10.00 | Nippon Gohshei and Various |
| Phenoxyethanol (Tech) | 0.70 | Various |
| Benzalkonium chloride 50% aqueous | 25.0 | Various |
| EDTA-4Na | 0.1 | Various |
| Deionised Water (qs to 100 %) | 64 | In-house |
| Total | 100.00 | |

This formulation is for application to a cellulosic substrate, which is for example, 40 g/m² paper. If two-ply paper is used, the dosage rate is doubled, and so on.

The actual dosage rate is calculated depending upon the degree of the anionic nature of the particular paper involved such that the paper can subsequently be used to achieve hospital grade hard surface disinfection according to the Australian TGA Option B under Dirty Conditions standard of antimicrobial performance.

The disinfection can be achieved in either of two ways. The surface to be disinfected can be sprayed with water and then the treated paper used to thoroughly wipe the surface clean.

Alternatively, the treated paper can be dampened with water prior to thoroughly wiping the surface clean.

In both cases only a very brief period of contact between the treated paper and the water involved is required to dissolve the active biocide which is then immediately available to perform its disinfection cycle.

The polyvinyl alcohol or other cationic compatible polymer is present simply to cause adhesion between the plys. The polymer also ensures that any residual biocide left on the surface, in this case a benzalkonium chloride, is not sticky or smeary but provides a tack free surface.

Of course, this same affect can be achieved with any other cationic biocide and in each case the correct dosage of the biocide to be applied to any cellulosic substrate must be calculated by experimentation before implementation.

### Formulation 2

Formulation 2 is a CHG composition, applied at a rate of 2.70 - 3.20 gm/m² paper to achieve at least 3 log (99.9%) reduction in both *E.coli* and *Staph aureus* using a standardised suspension test. Higher rates of dosage will achieve higher rates of kill.

| **Chemical name (active/grade)** | **Contents (w/w%)** | **Maker/Supplier** |
|---|---|---|
| Polyvinyl alcohol (Gohsenol GL05 Equivalent) | 13.00 | Nippon Gohsshei/Various |
| Polyethylene Glycol 400 (Tech) | 2.00 | Various |
| Phenoxyethanol (Tech) | 0.70 | Various |
| Chlorhexidine gluconate 20% w/v (Tech) | 53.50 | Various |
| Lactic Acid 88% (Tech) qs to pH -5.0 | -0.08 | Various |
| Deionised water Qs to 100% | -30.30 | In-house |
| Total | 100.00 | |

This formulation is for application to a cellulosic substrate e.g. Paper, 40 gm/m² (if two ply paper is used, the dosage rate is doubled, and so on) at a dosage rate measured in ml/m² with the dosage rate calculated depending upon the degree of the hydrophobic nature of the particular paper involved such that the paper can subsequently be used to achieve a measurable degree of bactericidal performance on skin.

The antisepsis is achieved by thoroughly wiping the hands dry after washing using the treated paper. Alternatively, the treated paper can be dampened with water prior to thoroughly wiping the hands or other areas of skin clean.

Different types of paper require different rates of addition of the treatment because each type has a different rate of deactivation of the cationic active because of different levels of anionic characteristics.

Once again, only a very brief period of contact between the treated paper and the water involved is required to dissolve the active biocide which is then immediately available to perform its disinfection cycle.

Of course, this same affect can be achieved with any other skin compatible, cationic biocide and the correct dosage of the biocide to be applied to any cellulosic substrate must be calculated by experimentation before implementation.

The formulation contains a proportion of polyethylene glycol. This additive is present both to accelerate the rate of solubility in water of the chlorhexidine gluconate (CHG) which is dry in the paper and also to improve skin feel after use of the treated paper.

The phenoxyethanol in the formulation is present to boost the antimicrobial activity of the CHG and the Lactic acid to adjust the pH of the formulation to optimise skin health.

Others example of a product which can be formulated using the principle disclosed in the invention is by the treatment of an appropriate cellulosic substrate such as the 2-ply paper towel described previously with a quaternary ammonium compound which produces beneficial skin effects. The liquid formulation would contain one or more of these quaternaries and could also contain components such as polyethylene glycol. This treated paper (or other cellulosic substrate), when used to dry the hands after washing would provide desirable skin feel properties. Of course, such a product may contain both a quaternary or quaternaries providing skin feel properties together with one or more quaternaries which are bactericidal, thus providing both a significant degree of antisepsis together with improved skin feel. Examples of those follow:

### Formulation 3

Formulation 3 is a skin conditioning composition that provides a reduction in the amount of Microorganisms present on the skin. Again, it is useful as a glue to adhere two plys of cellulosic material to one another.

| **Chemical name (active/ grade)** | **Contents (w/w)%** |
|---|---|
| Polyvinyl alcohol | 13.00 |
| Glycerine | 2.00 |
| Dimethyl silicone emulsion | 4.00 |
| Polysorbate 60 | 2.00 |
| Quaternium - 80 | 2.00 |
| Aloe juice 1:1 | 1.00 |
| Phenoxyethanol | 1.00 |
| Lactic acid | q.s. to pH -5.5 |
| Deionised Water | q.s to 100 ( ~75.0) |
| Total | 100.00 |

The various skin conditioning agents release while drying wet hands/ skin. The Aloe juice provides a soothing effect. A moisturising effect is achieved with glycerine and lactic acid, while emollient and skin feel effect are achieved with dimethyl silicone emulsion and quaternium - 80, which also acts as the cationic agent to reduce microbial load on the hands.

### Formulation 4

Formulation 4 is a skin conditioning composition that provides a reduction in the amount of Microorganisms present on the skin. Again it is useful as a glue to adhere two plys of cellulosic material.

| **Chemical name (active/ grade)** | **Contents (w/w)%** |
|---|---|
| Polyvinyl alcohol | 10.00 |
| Glycerine | 2.00 |
| PEG-6 Caprylic/Capric Glycerides | 2.00 |
| Isostearyl Palmitate | 1.50 |
| PEG-4 Laurate | 2.00 |
| Polysorbate 60 | 1.50 |
| Phenoxyethanol | 1.00 |
| Cyclopentasiloxane | 1.00 |
| Dimethyl silicone emulsion | 2.00 |
| Quaternium - 80 | 1.00 |
| Tocopheryl Acetate | 0.10 |
| Aloe juice 1:1 | 1.00 |
| Lactic acid | q.s. to pH ~5.5 |
| Deionised Water | q.s to 100 (~ 74.9) |
| Total | 100.00 |

The various skin conditioning agents release while drying wet hands/ skin. The Aloe juice provides a soothing effect. Tocopheryl Acetate provides a skin repair effect. A moisturising effect is achieved with glycerine and lactic acid, while emollient and skin feel effect are achieved with organo PEG glycerides, cyclopentasiloxanedimethyl silicone emulsion and quaternium - 80, which also acts as the cationic agent to reduce microbial load on the hands.

### Formulation 5

Another formulation within the scope of the present invention utilises formulas 1 or 2 but replaces the benzalkonium chloride or chlorhexidine gluconate in full or in part with di tallow esters, such as di-tallow ester of methyltriethanolammonium methosulfate, di-tallow ester of dimethyldiethanolammonium chloride, or di-tallow ester of trimethyl-dihydroxypropylammonium chloride. These formulations are useful in imparting fabric softening properties in wash. Impregnated cellulosic sheets can thus be made that can be included in washing machines to impart fabric softening and, when combined with a strong biocidal quaternary compound, can impart high levels of cleanliness to the washed clothing. The present invention provides a useful way to dispense an accurate dose of fabric softener and biocide to the wash. This is important to ensure that no more than the absolute minimum amount of fabric softener is dispensed into the waste water. The substrate can be multi-ply, with PVOH present as an adhesive, or it can be a relatively digestible substrate, such as lose weave tissue paper or multi-ply or coated tissue sheets.

### Cationic molecules

Examples of some of the active cationic molecules which can be used singly or in combination in the invention described are:
Group I: The alkyl substituted Quaternary ammonium compounds.
Group II: The non-halogenated benzyl substituted Quaternary ammonium compounds (including ethylbenzyl hydroxybenzyl, hydroxyethylbenzyl, naphthylmethyl, dodecylbenzyl, and alkyl benzyl)
Group III: The di- and tri-chlorobenzyl substituted quaternary ammonium compounds
Group IV: Quaternary ammonium compounds with unusual substitutes (charged heterocyclic compounds)
Group V Polymeric quaternary ammonium compounds (copolymerised with acrylamide and its derivatives) or vinyl pyrrolidone
Group VI Ester quaternary ammonium compounds formed by inserting an ester group in two of the alkyl chains
Group VII Biguanides such as chlorhexidine and octenidine salts
Groups I and II may be characterised by the formula R₁R₂R₃R₄ N⁺Anion e.g. Cl⁻,Br⁻ etc.

Where R₁R₂R₃R₄ are alkyl or benzyl or ethylbenzyl groups with alkyl groups having a chain length of from Csto C₁₈ and where typically R₁ is alkyl and R₂ benzyl or ethylbenzyl and R₃ and R₄ are methyl groups. Commercial examples of Groups I and II which are often a blend of Quaternary ammonium compounds of these types are:

| | |
|---|---|
| Bardac^{®} 2280 | Didecyldimethylammonium chloride |
| Bardac^{®} 2280i | Didecyldimethylammonium chloride |
| Bardac^{®} 2080 | Octyldecyldimethylammonium chloride |
| Bardap^{®} 26 | Didecylmethylpoly(oxyethyl)-ammonium propionate |
| Barquat^{®} BAG 50 | Alkyl (C12-C16) dimethylbenzylammonium chloride |
| Barquat^{®} BAG 80 | Alkyl (C12-C16] dimethylbenzylammonium chloride |
| Barquat^{®} CB50 | Alkyl (C12-C18) dimethylbenzylammonium chloride |
| Barquat^{®} CB80 | Alkyl (C12-C18) dimethylbenzylammonium chloride |
| Barquat^{®} DM50 | Alkyl (C12-C16) dimethylbenzylammonium chloride |
| Barquat^{®} DM50EP | Alkyl (C12-C16) dimethylbenzylammonium chloride |
| Barquat^{®} DM80 | Alkyl (C12-C16) dimethylbenzylammonium chloride |
| Barquat^{®} LB50 | Alkyl [C12-C14) dimethylbenzylammonium chloride |
| Barquat^{®} MB50 | Alkyl (C12-C16) dimethylbenzylammonium chloride |
| Barquat^{®} MB50W | Alkyl (C12-C16) dimethylbenzylammonium chloride |
| Barquat^{®} MB80 | Alkyl (C12-C16) dimethylbenzylammonium chloride |
| Barquat^{®} MS100 | Alkyl (C12-C14) dimethylbenzylammonium chloride |
| Barquat^{®} 4250-Z | Alkyl (C12-C18) dimethylbenzylammonium chloride and Alkyl (012-014) dimethylethylbenzylammonium chloride |

The following generic descriptions are applicable to quaternary ammonium compounds in Groups I - VI

| | |
|---|---|
| PREFERRED NAME | SYNONYM |
| Alkyl ethyl benzyl dimethyl ammonium chloride | |
| Aralkonium chloride | Alkyl dimethyl-3, 4-dichlorobenzyl ammonium chloride |
| Benzalkonium chloride | Alkyl dimethyl benzyl ammonium chloride |
| Cetalkonium chloride | Cetyl dimethyl benzyl ammonium chloride |
| Didecyl dimethyl ammonium chloride | Chloride didecyl dimethylammonium |
| Dioctyl dimethyl ammonium chloride | Chloride dioctyl dimethylammonium |
| Hexadecyl dimethyl benzyl ammonium chloride | Chloride hexadecyldimethylbenzyl ammonium |
| Methyl dodecyl benzyl trimethyl ammonium chloride | Chloride methyl dodecyl benzyl trimethyl ammonium |
| Octa decyl dimethyl benzyl ammonium chloride | Chloride octadecyl dimethylbenzyl ammonium |
| Octyl decyl dimethyl ammonium chloride | Chloride octyl decyl dimethyl ammonium |
| Octyl dimethyl ammonium chloride | Chloride octyl dimethyl ammonium |
| Benzalkonium Chloride CAS 8001-54-5 | |
| Benzethonium Chloride CAS 121-54-0 | |
| Cetalkonium Chloride CAS 122-18-9 | |
| Cetrimide CAS 8044-71-1 | |
| Cetrimonium Bromide CAS 57-09-0 | |
| Cetylpyridinium Chloride CAS 123-03-5 | |
| Glycidyl Trimethyl Ammonium Chloride CAS 3033-77-0 | |
| Diisobutylphenoxyethoxyethyldimethylbenzylammonium chloride; CAS 121-54-0 | |
| Benzylhexadecyldimethylammonium chloride; CAS 122-18-9 | |
| Cetyldimethylbenzylammonium chloride | |
| Hexadecyldimethylbenzylammonium chloride; | |
| Trimethyltetradecylammonium bromide | |
| Hexadecyltrimethylammonium bromide | |
| Hexadecyltrimethylammonium bromide | |
| Cetyltrimethylammonium bromide | |
| 1-Hexadecylpyridinium chloride; | |
| (2,3-epoxypropyl) trimethylammonium chloride | |
| Stearyldimethylbenzylammonium chloride CAS 122-19-0 | |
| Octenidine dihydrochloride CAS 79775-73-6 | |
| Chlorhexidene gluconate CAS 55-56-1 | |

### Group V Polymeric Quaternary ammonium compounds

This group are generally used in personal care products such as skin care products and hair care to enhance skin feel and hair manageability. They are generally inferior to compounds in Groups I, II,III, IV and VII with respect to antimicrobial action and are rarely used for that purpose.

### POLYQUATS (Polyquaternium Compounds)

Polyquaternium-1 Ethanol, 2,2',2 '' -nitrilotris-, polymer with 1,4-dichloro-2-butene and N,N,N',N'-tetramethyl-2-butene-1,4-diamine
Polyquaternium-2 Poly[bis(2-chloroethyl) ether-alt-1,3-bis[3-(dimethylamino)propyl]urea]
Polyquaternium-4 Hydroxyethyl cellulose dimethyl diallylammonium chloride copolymer; Diallyldimethylammonium chloride-hydroxyethyl cellulose copolymer
Polyquaternium-5 Copolymer of acrylamide and quaternized dimethylammoniumethyl methacrylate
Polyquaternium-6 Poly(diallyldimethylammonium chloride)
Polyquaternium-7 Copolymer of acrylamide and diallyldimethylammonium chloride
Polyquaternium-8 Copolymer of methyl and stearyl dimethylaminoethyl ester of methacrylic acid, quaternized with dimethylsulphate[2]
Polyquaternium-9 Homopolymer of N,N-(dimethylamino)ethyl ester of methacrylic acid, quaternized with bromomethane
Polyquaternium-10 Quaternized hydroxyethyl cellulose
Polyquaternium-11 Copolymer of vinylpyrrolidone and quaternized dimethylaminoethyl methacrylate
Polyquaternium-12 Ethyl methacrylate / abietyl methacrylate / diethylaminoethyl methacrylate copolymer quaternized with dimethyl sulfate
Polyquaternium-13 Ethyl methacrylate / oleyl methacrylate / diethylaminoethyl methacrylate copolymer quaternized with dimethyl sulfate
Polyquaternium-14 Trimethylaminoethylmethacrylate homopolymer
Polyquaternium-15 Acrylamide-dimethylaminoethyl methacrylate methyl chloride copolymer
Polyquaternium-16 Copolymer of vinylpyrrolidone and quaternized vinylimidazole
Polyquaternium-17 Adipic acid, dimethylaminopropylamine and dichloroethylether copolymer
Polyquaternium-18 Azelaic acid, dimethylaminopropylamine and dichloroethyl ether copolymer
Polyquaternium-19 Copolymer of polyvinyl alcohol and 2,3-epoxypropylamine
Polyquaternium-20 Copolymer of polyvinyl octadecyl ether and 2,3-epoxypropylamine
Polyquaternium-22 Copolymer of acrylic acid and diallyldimethylammonium Chloride
Polyquaternium-24 Quaternary ammonium salt of hydroxyethyl cellulose reacted with a lauryl dimethyl ammonium substituted epoxide.
Polyquaternium-27 Block copolymer of Polyquaternium-2 and Polyquaternium-17
Polyquaternium-28 Copolymer of vinylpyrrolidone and methacrylamidopropyl trimethylammonium
Polyquaternium-29 Chitosan modified with propylene oxide and quaternized with epichlorhydrin
Polyquaternium-30 Ethanaminium, N-(carboxymethyl)-N,N-dimethyl-2-[(2-methyl-1-oxo-2-propen-1-yl)oxy]-, inner salt, polymer with methyl 2-methyl-2-propenoate
Polyquaternium-31 N,N- dimethylaminopropyl-N-acrylamidine quaternized with diethylsulfate bound to a block of polyacrylonitrile
Polyquaternium-32 Poly(acrylamide 2-methacryloxyethyltrimethyl ammonium chloride)
Polyquaternium-33 Copolymer of trimethylaminoethylacrylate salt and acrylamide
Polyquaternium-34 Copolymer of 1,3-dibromopropane and N,N-diethyl-N',N'-dimethyl-1,3-propanediamine
Polyquaternium-35 Methosulphate of the copolymer of methacryloyloxyethyltrimethylammonium and of methacryloyloxyethyldimethylacetylammonium
Polyquaternium-36 Copolymer of N,N-dimethylaminoethylmethacrylate and buthylmethacrylate, quaternized with dimethylsulphate
Polyquaternium-37 Poly(2-methacryloxyethyltrimethylammonium chloride)
Polyquaternium-39 Terpolymer of acrylic acid, acrylamide and diallyldimethylammonium Chloride
Polyquaternium-42 Poly[oxyethylene(dimethylimino)ethylene (dimethylimino)ethylene dichloride]
Polyquaternium-43 Copolymer of acrylamide, acrylamidopropyltrimonium chloride, 2-amidopropylacrylamide sulfonate and dimethylaminopropylamine
Polyquaternium-44 3-Methyl-1-vinylimidazolium methyl sulfate-N-vinylpyrrolidone copolymer
Polyquaternium-45 Copolymer of (N-methyl-N-ethoxyglycine)methacrylate and N,N-dimethylaminoethylmethacrylate, quaternized with dimethyl sulphate
Polyquaternium-46 Terpolymer of vinylcaprolactam, vinylpyrrolidone, and quaternized vinylimidazole
Polyquaternium-47 Terpolymer of acrylic acid, methacrylamidopropyl trimethylammonium chloride, and methyl acrylate

### Group VI Ester Quaternary Ammonium Compounds

Typical applications for this group are in formulating Laundry fabric softeners providing mild antimicrobial action. For increased antimicrobial performance products from Groups I and II may be added.

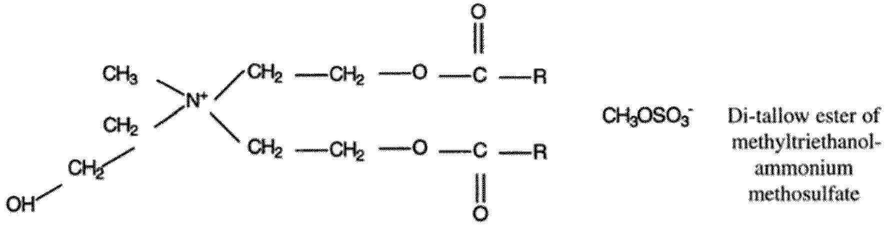

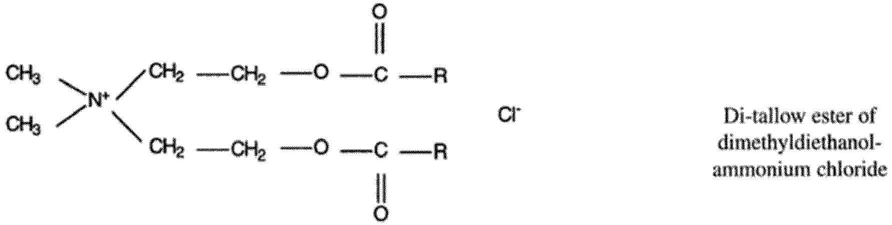

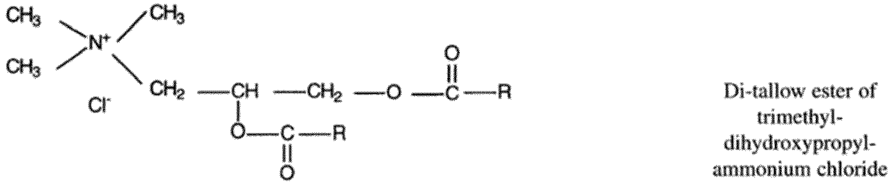

Commercially available examples include the TETRANYL ^{®} REWOQUAT^{®} and ACCOSOFT^{®} Product Ranges from Kao Corp Evonik Industries and Stepan Chemical respectively

### Group VII Biguanides

Chlorhexidine Gluconate (CHG) 1,6-bis(4-chloro-phenylbiguanido)hexane CAS 55-56-1,
Octenidine Hydrochloride N,N'-(decane-1,10-diyldipyridin-1-yl-4-ylidene)dioctan-1-amine Dihydrochloride CAS 70775-73-6,
Polyhexanide PHMB Polyhexamethylene biguanide CAS 28757-47-3, Example brand name VANTOCIL ^{®} Lonza Corp

### Water-Soluble Polymers

Examples of some of the water-soluble polymers, dispersions or emulsions which can be used in the invention described are:

### Synthetic Water-Soluble Polymers

Poly(ethyleneglycol) PEG
Poly(propyleneglycol) PPG
Poly(vinylpyrrolidone) PVP
Poly(vinylpyrrolidone/vinylacetate) copolymer PVP/VA
Poly(vinylpyrrolidone/dimethylaminoethylmethacrylate Copolymer PVP/DMAEMA
Poly(acrylicacid) PAA
Poly(oxyethylene/oxypropylene) glycol copolymers
Sodium Polyacrylate solutions
Ammonium Polyacrylate solutions
Poly (dimethylsiloxane) emulsions
Poly(vinylalcohol) PVOH
Styrene/Butadiene Copolymer emulsion
Carboxylated Styrene/Butadiene copolymer emulsion
Alkyl methacrylate copolymer emulsion
PolyAcrylic Acid emulsion
Acrylic Acid/VinylAcetate emulsion
Polyvinyl Acetate emulsions
Polyethylene emulsion cationic and nonionic
Epoxy ester polymer emulsion
Polyurethane polymer dispersion
Polystyrene polymer dispersion
AlkoxyAlkyl substituted Polyacrylicx Acid R¹OH/R² PAA
Divinylether/MaleicAnhydride copolymers DIVEMA
Poly(vinylether/maleic Anhydride) PVM/MA
Poly(vinylether/maleic Acid) PVM/MA
Poly (ethyleneimine)
Quaternary Polyamines
Poly(diallyldimethylammonium chloride PDADMAC
Poly (2-Alkyl-2-Oxazoline)
Poly[di(carboxylatophenoxy)phosphazene] (PCPP)
Poly[di(methoxyethoxyethoxy) phosphazene] (MEEP)
Methyl cellulose
Hydroxyethylcellulose (HEC)
Hydroxypropylcellulose (HPC)
Hydroxypropylmethyl cellulose (HPMC)
Sodium Carboxymethyl Cellulose (Na CMC)

### Natural Water-Soluble Polymers

Xanthan Gum
Pectin
Hyaluronic Acid
Chitosan
Dextran
Partially Hydrolysed Starch - Maltodextrin
Carrageenan
Guar Gum

### Skin Conditioning Agents

Examples of some of the active skin conditioning agents (along with their skin effect) which can be used singly or in combination in the invention described are:

| **INCI name** | **Skin effect** |
|---|---|
| Silicone Quaternium-20 | Skin conditioning agent for mildness, silky feel and smoothness. |
| Silicone Quaternium-24 | Skin conditioning agent for mildness, silky feel and smoothness. |
| Quarternium-45 (3,4-Dimethyl-2-[2-(phenylamino)vinyl]oxazolium iodide) | Skin moisturising, repairing agent |
| POLYQUATERNIUM-10 | Skin conditioning, moisturising agent Silky feel |
| POLYQUATERNIUM-50 | Skin conditioning agent for silky feel and smoothness. |
| QUATERNIUM-79 HYDROLYZED WHEAT PROTEIN | Skin conditioning |
| Cocamidopropyl ethyldimonium ethosulfate | Skin soft after-feel and moisturizing effects |
| GUAR HYDROXYPROPYLTRIMONIUM CHLORIDE | Skin conditioning, moisturising agent |
| STARCH HYDROXYPROPYLTRIMONIUM CHLORIDE | Skin moisturising agent. Silky feel |
| POLYMETHACRYLAMIDOPROPYLTRIMONIUM CHLORIDE | Skin moisturising agent. Silky feel |
| PEG-2 DIMEADOWFOAMAMIDOETHYLMONIUM METHOSULFATE | Skin conditioning, moisturising agent |
| SILICONE QUATERNIUM-2 PANTHENOL SUCCINATE | Skin conditioning |

We have discussed use of the invention for disinfecting hard surfaces such as in hospitals and in Kitchens, for use in a novel paper towel which not only dries hands after washing but leaves them bacteriostatic without the need for separate application of antimicrobial creams and the like, and for imparting skin feel improvers.

Other uses for the invention include the preparation of curtains for use in hospitals for example curtains surrounding bed cubicles for privacy as well as window covering curtains. Because of high patient turnover in cubicles, and the need for frequent handling of the curtain for opening and closing by a variety of patients, visitors, medical staff and orderlies, cubical surrounding privacy curtains are at high risk of conveying crossinfecting microbes and hence require frequent disinfection. Paper curtains treated according to the present invention remain biostatic for long periods and can then be disposed of at much lower materials and labour cost than the repetitively cleaning/disinfecting regimes currently used. A further potential application of the invention is to treat cardboard for the manufacture of cardboard so that cartons manufactured from the treated cardboard is resistant to mould growth enabling the manufacture of mould resistant cardboard boxes.

Those skilled in the art will appreciate that the invention can be beneficially used in many other ways

### TEST METHOD FOR ANTISEPTIC PAPER WIPE VALIDATION

This test method was published in the "Australian Journal of Hospital Pharmacy", Vol 8, No 4; 1978 (152-155)

### 1. Principle

The method, as applied to Hospital Grade Disinfectants or Sanitisers, is essentially that given by Kelsey & Maurer (1) for testing disinfectant performance. It is set out in a form suitable for attachment to a regulatory minimum standard for disinfectants and antiseptics. For wider application of the test refer to supplementary note A.

The disinfectant is tested at the dilution recommended by the manufacturer on the product label. The test consists of challenging the diluted disinfectant with bacterial inoculum, withdrawing a sample after a given time and culturing the sample in a suitable recovery medium. After this sampling, the mixture is again challenged by a second inoculum and after a second interval is again sampled for culturing. The sample is passed or failed according to the extent of growth shown in the two cultures sampled. The test may be performed with or without the addition of sterile yeast as an organic soil. (Options B and A respectively) or both, according to the use-situations advocated on the label of the product under test.

**Table 1. Selection of test parameters for classes of disinfectant and antiseptic using the TGA Disinfectant Test.**

| Class of product | Organisms used in the test | Test option for resuspension of centrifuged organisms | Number of challenges | Inoculum density |
|---|---|---|---|---|
| Disinfectant - hospital grade: Sanitiser | *Ps. aeruginosa* | A ("clean" conditions) | 2 | 2×10⁸ - 2×10⁹ |
| | *Pr. vulgaris* | | | |
| | *E. coli* | B ("dirty" conditions") | | |
| | *S. aureus* | | | |
| Disinfectant - household or commercial grade | *E. coli* | C | 1 | 2×10⁸ - 2×10⁹ |
| | *S. aureus* | | | |
| Antiseptic (excluding those for intact skin only) | *Ps. aeruginosa* | D | 1 | 1×10⁶ -1×10⁷ |
| | *Pr. vulgaris* | | | |
| | *E. coli* | | | |
| | *S. aureus* | | | |

For Household Grade disinfectants, the first two organisms listed and the second challenge are omitted, while Option C (nutrient broth) is selected as the choice of simulated soil. For antiseptics, the second challenge is again omitted, while Option D (serum) is selected as the choice of soil.

### 2. Media

All media must be contained in capped glass containers. Where media are stored, the containers must be sealed tightly or refrigerated.

### 2.1 Sterile Hard Water

2.1.1 Dissolve 0.304g anhydrous calcium chloride and 0.065g anhydrous magnesium chloride in glass-distilled water, and make up to one litre.
2.1.2 Dispense into glass containers and sterilize by autoclaving at 121°±1°C for 15 minutes.

### 2.2 Yeast Suspension

2.2.1 Weigh 200g of moist compressed baker's yeast. Cream by the gradual addition of sterile hard water using a heavy glass rod for stirring. Decant the creamed portion into a flask, add more water to any lumpy residue remaining and repeat the creaming and decantation until no residue remains and 500ml of water has been used.
2.2.2 Shake the contents of the flask vigorously and strain through a 100-mesh sieve, breaking down any remaining lumps.
2.2.3 Add 500ml sterile hard water, shake vigorously and adjust the pH to 6.9-7.1 with 1N Sodium hydroxide.
2.2.4 Transfer 50ml, 100ml or 200ml of the yeast solution into screw-capped bottles.
2.2.5 Autoclave at 121°±1°C for 15 minutes and allow the autoclave to cool without releasing pressure. Store cold but not freezing.
2.2.6 Dry two Petri dishes to constant weight. Into each, pipette 25ml of sterilised yeast suspension, and dry to constant weight at 100°C. Calculate the average solids content of the suspension.
2.2.7 Before use, pipette 25ml of the sterilised yeast suspension into a beaker. Determine the pH using the glass electrode, and determine the volume of 1N sodium hydroxide solution needed to adjust the pH to within the range 6.9 to 7.1.
2.2.8 Immediately before use, add to each bottle of sterilised yeast, a volume of sterile hard water and a volume of 1N sodium hydroxide calculated to adjust the concentration of dry yeast to 5.0% and the pH to within the range 6.9-7.1. Discard prepared yeast 3 months after preparation.

### 2.3 Medium for Growth of Test Organisms

2.3.1 Prepare a 10% w/v dextrose solution in distilled water, and sterilise by autoclaving at 121°±1°C for 15 minutes. Cool to room temperature.
2.3.2 Prepare Wright and Mundy medium following the author's procedure (2) or from a commercial product of the same composition (Note B) and sterilise by autoclaving at 121°±1°C for 15 minutes. Cool to room temperature.
2.3.3 To each litre of Wright and Mundy medium prepared in 2.3.2 add 10ml sterile dextrose solution prepared in 2.3.1.
2.3.4 Aseptically dispense in either 10ml or 15ml amounts, as preferred.
2.3.5 This medium is referred to as Wright and Mundy dextrose medium.

### 2.4 Recovery Medium

2.4.1 Prepare nutrient broth as follows or from a commercial product of the same composition

### (Note B):-

| | |
|---|---|
| Add the following to 970ml of water and dissolve by heating. Beef Extract Powder | 10g |
| Peptone | 10g |
| Sodium Chloride | 5g |

| | |
|---|---|
| Adjust the pH to 8.0-8.4 using 1N Sodium Hydroxide. Boil for 10 minutes and filter. Cool. | |

2.4.2 To each litre of nutrient broth solution prepared in 2.4.1 add 30g polysorbate 80 (Note B).

2.4.3 Adjust pH to 7.2-7.4, using 1N Sodium hydroxide.

2.4.4 Autoclave at 121°±1°C for 15 minutes, and immediately shake well to disperse the polysorbate 80.

2.4.5 Dispense aseptically in 10ml amounts into sterile capped glass tubes.

### 3. Test Inoculum

### 3.1 Test Organisms

The following 4 organisms are to be used, except where prescribed.

| | |
|---|---|
| *Pseudomonas aeruginosa* | NCTC 6749 |
| *Proteus vulgaris* | NCTC 4635 |
| *Escherichia coli* | NCTC 8196 |
| *Staphylococcus aureus* | NCTC 4163 |

### 3.2 Preparation of Inoculum

3.2.1 Incubate the contents of an ampoule of freeze-dried culture overnight at 37°±1°C in Wright and Mundy dextrose medium.

3.2.2 Inoculate the incubated culture onto nutrient agar slopes in McCartney bottles. Store for up to 3 months at 4°±1°C.

3.2.3 At a suitable period before the test is to be conducted, sub-culture from an agar slope into 10ml or 15ml quantities of Wright and Mundy dextrose medium. Incubate at 37°±1°C for 24±2 hours.

3.2.4 Sub-culture from the medium in 3.2.3 into fresh medium, using an inoculating loop of 4mm in diameter. Incubate at 37°±1°C for 24±2 hours.

3.2.5 Repeat step 3.2.4 daily. For the test procedure use only those cultures which have been sub-cultured at least 5, and not more than 14 times.

3.2.6 Filter test cultures of P. aeruginosa and S. aureus through sterile Whatmans No. 4 filter paper.

3.2.7 Centrifuge all test cultures until cells are compact, and remove supernatant with a Pasteur pipette.

3.2.8 Resuspend test organisms in the original volume of liquid (i.e. 10ml or 15ml), and shake for 1 minute with a few sterile glass beads.

3.2.8.1 For Option A, resuspend in sterile hard water.

3.2.8.2 For Option B, resuspend in a mixture of 4 parts yeast suspension (prepared as in 2.2) to 6 parts sterile hard water.

3.2.8.3 For Option C, resuspend in nutrient broth (prepared as in 2.4.1 and 2.4.3 and sterilised by autoclaving).

3.2.8.4 For Option D, resuspend in sterile hard water; dilute twice 1 + 9 in sterile hard water; then add 8ml of the last dilution to 2ml sheep serum previously inactivated at 56°C for 20 mins. and sterilised by filtration.

### 3.3 Enumeration of Inoculum

Immediately before testing, sample the resuspended inoculum and enumerate using 10-fold dilutions in quarter-strength Ringer's solution and the pour-plate technique. The number subsequently counted must represent not less than 2 × 10⁸ or more than 2 × 10⁹ organisms per millilitre (or 1 × 10⁸ - 1 × 10⁷ using Option D) or the test is considered invalid. Retain tube containing 10⁻⁷ dilution for use in controls (7.3 and 7.4).

### 4. Disinfectant Dilutions

Quantitatively dilute a sample of the disinfectant to the specified extent, using sterile hard water as diluent. Use not less than 10ml or 10g of sample for the first dilution, and not less than 1 ml of any dilution to prepare subsequent dilutions. Make all dilutions in glass containers on the day of testing. The glass containers must be twice rinsed in glass-distilled water, and sterilised.

### 5. Temperature

Where air-conditioning does not maintain test solutions at 21°±1°C, hold the containers in which the test is to be carried out in a waterbath at this temperature.

### 6. Test Procedure

Perform the following test using each of the four test organisms (3.1) except where the Standard directs otherwise. It is not necessary to test with all organisms simultaneously.

6.1 Add 3ml of diluted disinfectant to a capped glass container.

6.2 Start a timing device. Immediately inoculate disinfectant with 1ml of culture (prepared in 3.2) and mix by swirling.

6.3 At 8 minutes, subculture one drop (0.02ml + .002ml) into each of 5 tubes containing recovery broth. To ensure delivery of 0.02ml into the first tube of recovery broth at exactly 8 minutes, it will be necessary to withdraw a suitable amount from the disinfectant test mix shortly beforehand. This must be immediately preceded by vortexing. Surplus sample must be returned to the test mix. (See Note D).

6.4 Except where prescribed, at 10 minutes, inoculate disinfectant with a further 1ml of culture, and mix by vortexing.

6.5 Except where prescribed, at 18 minutes, proceed as in 6.3.

6.6 Mix the contents of all tubes of recovery broth by vortexing. Incubate at 37°±1°C for 48±2 hours.

6.7 Examine for growth and record results.

6.8 For each test organism repeat steps 6.1-6.7 on each of 2 subsequent days, using a fresh disinfectant dilution and a freshly prepared bacterial suspension.

### 7. Controls

### 7.1 Recovery broth contamination

Incubate one uninoculated tube of recovery broth at 37°±1°C for 48±2 hours and examine for growth. If growth occurs, the test is considered invalid due to contamination of the recovery broth.

### 7.2 Disinfectant contamination

To 1 tube of recovery broth, add 0.02ml of diluted disinfectant. Incubate at 37°±1°C for 48±2 hours. If growth occurs, the test is considered invalid. Growth in 7.2 but not 7.1 indicates contamination of the disinfectant test solution.

### 7.3 Fertility Test

To 1 tube of recovery broth, add 1.0ml of the 10⁻⁷ dilution retained in 3.3. Incubate at 37°±1°C for 48±2 hours and examine for growth. If no growth occurs, the test is considered invalid.

### 7.4 Inactivator Efficacy

To 1 tube of recovery broth, add 0.02ml of diluted disinfectant and 1.0ml of the 10⁻⁷ dilution retained in 3.3. Incubate at 37°±1°C for 48±2 hours, and examine for growth. If no growth occurs, the test is considered invalid. Growth in 7.3 but not in 7.4 indicates inadequate inactivation of the disinfectant.

### 8. Procedure in case of invalid controls

When any control renders the test invalid, the test is to be repeated. Fresh recovery broth is to be used if growth occurred in control 7.1 or if no growth occurred in controls 7.3 or 7.4.

Should disinfectant contamination be indicated by control 7.2 on both occasions, the disinfectant is considered to fail the test. Should inadequate inactivation of the disinfectant be indicated by control 7.4 on both occasions, the test is considered invalid (Note C).

### 9. Results

The dilution test passes the test if there is no apparent growth in at least two out of the five recovery broths specified in 6.3 and no apparent growth in at least two of the five recovery broths specified in 6.5 on all three occasions, using all four organisms.

### 10. References

(1) Kelsey, J.C. and Maurer Isobel, M. Pharmaceutical Journal (UK) 213: 528-530, (1974). (2) Wright Eleanore, S. and Mundy, R.A. Journal of Bacteriology 80: 279-280, (1960).

## Claims

1. An applicator for providing an active composition to a surface, the applicator comprising at least two layers of cellulosic substrate having anionic sites;
said layers being adhered to each other by an adhesive composition, the adhesive composition comprising a cationic component in an amount sufficient in use to deactivate the anionic sites in the cellulosic substrate and to provide a desired effect, wherein the desired effect is selected from one or more of a biocidal effect, enhanced skin feel, enhanced skin health and fabric softening;
wherein the adhesive composition also comprises a water-soluble or water dispersible adhesive polymer;
wherein the water-soluble or water-dispersible adhesive polymer is selected from one or more of PVOH, polyvinyl pyrrolidone or copolymers of polyvinyl pyrrolidone with vinyl acetate, non-ionic polyvinyl acetate emulsions or vinyl ether/maleic anhydride copolymer; and
wherein the cationic component is a cationic biocide or a skin compatible cationic selected from benzalkonium chloride, benzethonium chloride, alkylammonium chloride, chlorhexidine gluconate, octenidine hydrochloride, tallow quaternary compounds or silicone-based quaternaries.

2. An applicator according to claim 1 wherein each one of said at least two layers are the same.

3. An applicator according to claim 1 wherein each one of said at least two layers are different.

4. An applicator according to any one of claims 1 to 3 comprising multiple layers of cellulosic substrate adhered to each other by an adhesive composition.

5. An applicator according to any one of the preceding claims wherein the cationic component is a cationic biocide, selected from the group consisting of a quaternary ammonium compound.

6. An applicator according to any one of claims 1 to 5, wherein the cationic component is a skin compatible cationic selected from benzalkonium chloride, benzethonium chloride, alkylammonium chloride, chlorhexidine gluconate, octenidine hydrochloride, tallow quaternary compounds or silicone-based quaternaries.

7. An applicator according to any one of the preceding claims wherein the cellulosic substrate is selected from the group consisting of paper, paper laminate, cardboard, a plant fabric or the like, optionally, wherein the plant fabric is a wood pulp or cotton material, and optionally, wherein the cellulosic substrate is woven, or wherein the cellulosic substrate is non-woven.

8. An applicator according to any one of the preceding claims which is substantially dry.

9. A method of making an applicator, the method comprising:
• contacting the surface of a first ply of cellulosic material with an adhesive composition comprising a cationic component, and
• applying to the adhesive composition a further ply of cellulosic material,
wherein the adhesive composition also comprises a water-soluble or water dispersible adhesive polymer, wherein the water-soluble or water-dispersible adhesive polymer is selected from one or more of PVOH, polyvinyl pyrrolidone or copolymers of polyvinyl pyrrolidone with vinyl acetate, non-ionic polyvinyl acetate emulsions or vinyl ether/maleic anhydride copolymer; and
wherein the cationic component is a cationic biocide or a skin compatible cationic selected from benzalkonium chloride, benzethonium chloride, alkylammonium chloride, chlorhexidine gluconate, octenidine hydrochloride, tallow quaternary compounds or silicone-based quaternaries; and
wherein the cationic component is present in an amount sufficient in use to deactivate the anionic sites in the cellulosic substrate and to provide a desired effect, wherein the desired effect is selected from one or more of a biocidal effect, enhanced skin feel, enhanced skin health and fabric softening.

10. A method of disinfecting an inanimate surface, the method comprising moistening the surface, and then wiping it with an applicator according to any one of claims 1 to 8.

11. A method of disinfecting an inanimate surface, the method comprising moistening an applicator according to any one of claims 1 to 8, and then wiping the surface with the moistened applicator, wherein the applicator comprises paper or fabric, and whereby to apply a bioactive cationic species to the surface.

12. A method of disinfecting skin, the method comprising moistening the skin and then wiping it with an applicator according to any of claims 1 to 8.

13. A method of disinfecting skin, the method comprising moistening an applicator according to any one of the claims 1 to 8, and then wiping the skin with the moistened applicator, wherein the applicator comprises paper or fabric, and whereby to apply a bioactive cationic species to the skin.

## Patentansprüche

1. Applikator zum Bereitstellen einer aktiven Zusammensetzung für eine Oberfläche, wobei der Applikator mindestens zwei Schichten aus Zellulosesubstrat umfasst, die anionische Stellen aufweisen;
wobei die Schichten durch eine Klebstoffzusammensetzung miteinander verklebt sind, wobei die Klebstoffzusammensetzung eine kationische Komponente in einer Menge umfasst, die bei Verwendung ausreicht, um die anionischen Stellen im Zellulosesubstrat zu deaktivieren und eine gewünschte Wirkung bereitzustellen, wobei die gewünschte Wirkung aus einem oder mehreren von einer bioziden Wirkung, einem verbesserten Hautgefühl, einer verbesserten Hautgesundheit und einer Weichmachung von Gewebe ausgewählt ist;
wobei die Klebstoffzusammensetzung auch ein wasserlösliches oder wasserdispergierbares Klebstoffpolymer umfasst;
wobei das wasserlösliche oder wasserdispergierbare Klebstoffpolymer aus einem oder mehreren von PVOH, Polyvinylpyrrolidon oder Copolymeren von Polyvinylpyrrolidon mit Vinylacetat, nichtionischen Polyvinylacetatemulsionen oder Vinylether- /Maleinsäureanhydrid-Copolymer ausgewählt ist; und
wobei die kationische Komponente ein kationisches Biozid oder ein hautverträgliches kationisches Mittel, ausgewählt aus Benzalkoniumchlorid, Benzethoniumchlorid, Alkylammoniumchlorid, Chlorhexidingluconat, Octenidinhydrochlorid, quaternären Talgverbindungen oder quaternären Verbindungen auf Silikonbasis ist.

2. Applikator nach Anspruch 1, wobei jede der mindestens zwei Schichten die gleiche ist.

3. Applikator nach Anspruch 1, wobei jede der mindestens zwei Schichten unterschiedlich ist.

4. Applikator nach einem der Ansprüche 1 bis 3, der mehrere Schichten aus Zellulosesubstrat umfasst, die durch eine Klebstoffzusammensetzung miteinander verklebt sind.

5. Applikator nach einem der vorstehenden Ansprüche, wobei die kationische Komponente ein kationisches Biozid, ausgewählt aus der Gruppe, die aus einer quaternären Ammoniumverbindung besteht, ist.

6. Applikator nach einem der Ansprüche 1 bis 5, wobei die kationische Komponente ein hautverträgliches kationisches Mittel, ausgewählt aus Benzalkoniumchlorid, Benzethoniumchlorid, Alkylammoniumchlorid, Chlorhexidingluconat, Octenidinhydrochlorid, quaternären Talgverbindungen oder quaternären Verbindungen auf Silikonbasis ist.

7. Applikator nach einem der vorstehenden Ansprüche, wobei das Zellulosesubstrat aus der Gruppe bestehend aus Papier, Papierlaminat, Pappe, einem pflanzlichen Gewebe oder dergleichen ausgewählt ist, wobei das pflanzliche Gewebe optional ein Zellstoff oder Baumwollmaterial ist und wobei das Zellulosesubstrat optional gewebt ist oder wobei das Zellulosesubstrat nicht gewebt ist.

8. Applikator nach einem der vorstehenden Ansprüche, der im Wesentlichen trocken ist.

9. Verfahren zum Herstellen eines Applikators, wobei das Verfahren umfasst:
- Inkontaktbringen der Oberfläche einer ersten Lage aus Zellulosematerial mit einer Klebstoffzusammensetzung, die eine kationische Komponente umfasst, und
- Aufbringen einer weiteren Lage aus Zellulosematerial auf die Klebstoffzusammensetzung,
wobei die Klebstoffzusammensetzung auch ein wasserlösliches oder wasserdispergierbares Klebstoffpolymer umfasst, wobei das wasserlösliche oder wasserdispergierbare Klebstoffpolymer aus einem oder mehreren von PVOH, Polyvinylpyrrolidon oder Copolymeren von Polyvinylpyrrolidon mit Vinylacetat, nichtionischen Polyvinylacetatemulsionen oder Vinylether-/Maleinsäureanhydrid-Copolymer ausgewählt ist; und
wobei die kationische Komponente ein kationisches Biozid oder ein hautverträgliches kationisches Mittel, ausgewählt aus Benzalkoniumchlorid, Benzethoniumchlorid, Alkylammoniumchlorid, Chlorhexidingluconat, Octenidinhydrochlorid, quaternären Talgverbindungen oder quaternären Verbindungen auf Silikonbasis ist; und
wobei die kationische Komponente in einer Menge vorhanden ist, die bei Verwendung ausreicht, um die anionischen Stellen im Zellulosesubstrat zu deaktivieren und eine gewünschte Wirkung bereitzustellen, wobei die gewünschte Wirkung aus einem oder mehreren von einer bioziden Wirkung, einem verbesserten Hautgefühl, einer verbesserten Hautgesundheit und einer Weichmachung von Gewebe ausgewählt ist.

10. Verfahren zum Desinfizieren einer unbelebten Oberfläche, wobei das Verfahren das Befeuchten der Oberfläche und dann das Abwischen derselben mit einem Applikator nach einem der Ansprüche 1 bis 8 umfasst.

11. Verfahren zum Desinfizieren einer unbelebten Oberfläche, wobei das Verfahren das Befeuchten eines Applikators nach einem der Ansprüche 1 bis 8 und dann das Abwischen der Oberfläche mit dem befeuchteten Applikator umfasst, wobei der Applikator Papier oder Gewebe umfasst, und wodurch eine bioaktive kationische Spezies auf die Oberfläche aufzubringen ist.

12. Verfahren zum Desinfizieren von Haut, wobei das Verfahren das Befeuchten der Haut und dann das Abwischen derselben mit einem Applikator nach einem der Ansprüche 1 bis 8 umfasst.

13. Verfahren zum Desinfizieren von Haut, wobei das Verfahren das Befeuchten eines Applikators nach einem der Ansprüche 1 bis 8 und dann das Abwischen der Haut mit dem befeuchteten Applikator umfasst, wobei der Applikator Papier oder Gewebe umfasst, und wodurch eine bioaktive kationische Spezies auf die Haut aufzubringen ist.

## Revendications

1. Applicateur pour disposer une composition active sur une surface, l'applicateur comprenant au moins deux couches de substrat cellulosique présentant des sites anioniques ;
lesdites couches étant collées les unes aux autres par une composition adhésive, la composition adhésive comprenant un composant cationique en une quantité suffisante lors de l'utilisation pour désactiver les sites anioniques dans le substrat cellulosique et pour fournir un effet souhaité, dans lequel l'effet souhaité est choisi parmi un ou plusieurs d'un effet biocide, d'une meilleure sensation sur la peau, d'une peau plus saine et d'un assouplissement de tissus ;
dans lequel la composition adhésive comprend également un polymère adhésif hydrosoluble ou dispersible dans l'eau ;
dans lequel le polymère adhésif soluble ou dispersible dans l'eau est choisi parmi un ou plusieurs parmi le PVOH, la polyvinylpyrrolidone ou des copolymères de polyvinylpyrrolidone avec de l'acétate de vinyle, des émulsions d'acétate de polyvinyle non ioniques ou un copolymère d'éther de vinyle/anhydride maléique ; et
dans lequel le composant cationique est un biocide cationique ou un cationique compatible avec la peau choisi parmi le chlorure de benzalkonium, le chlorure de benzéthonium, le chlorure d'alkylammonium, le gluconate de chlorhexidine, le chlorhydrate d'octénidine, les composés quaternaires de suif ou les quaternaires à base de silicone.

2. Applicateur selon la revendication 1 dans lequel chacune desdites au moins deux couches est identique.

3. Applicateur selon la revendication 1 dans lequel chacune desdites au moins deux couches est différente.

4. Applicateur selon l'une quelconque des revendications 1 à 3 comprenant plusieurs couches de substrat cellulosique collées les unes aux autres par une composition adhésive.

5. Applicateur selon l'une quelconque des revendications précédentes dans lequel le composant cationique est un biocide cationique, choisi dans le groupe consistant en un composé d'ammonium quaternaire.

6. Applicateur selon l'une quelconque des revendications 1 à 5, dans lequel le composant cationique est un cationique compatible avec la peau choisi parmi le chlorure de benzalkonium, le chlorure de benzéthonium, le chlorure d'alkylammonium, le gluconate de chlorhexidine, le chlorhydrate d'octénidine, les composés quaternaires de suif ou les quaternaires à base de silicone.

7. Applicateur selon l'une quelconque des revendications précédentes dans lequel le substrat cellulosique est choisi dans le groupe consistant en du papier, du papier stratifié, du carton, un tissu végétal ou analogue, facultativement, dans lequel le tissu végétal est une pâte de bois ou un matériau en coton, et facultativement, dans lequel le substrat cellulosique est tissé, ou dans lequel le substrat cellulosique est non tissé.

8. Applicateur selon l'une quelconque des revendications précédentes qui est sensiblement sec.

9. Procédé de fabrication d'un applicateur, le procédé comprenant :
- la mise en contact de la surface d'une première couche de matériau cellulosique avec une composition adhésive comprenant un composant cationique, et
- l'application à la composition adhésive d'une couche supplémentaire de matériau cellulosique,
dans lequel la composition adhésive comprend également un polymère adhésif hydrosoluble ou dispersible dans l'eau, dans lequel le polymère adhésif hydrosoluble ou dispersible dans l'eau est choisi parmi un ou plusieurs parmi le PVOH, la polyvinylpyrrolidone ou des copolymères de polyvinylpyrrolidone avec de l'acétate de vinyle, des émulsions d'acétate de polyvinyle non ioniques ou un copolymère d'éther de vinyle/anhydride maléique ; et
dans lequel le composant cationique est un biocide cationique ou un cationique compatible avec la peau choisi parmi le chlorure de benzalkonium, le chlorure de benzéthonium, le chlorure d'alkylammonium, le gluconate de chlorhexidine, le chlorhydrate d'octénidine, les composés quaternaires de suif ou les quaternaires à base de silicone ; et
dans lequel le composant cationique est présent en une quantité suffisante lors de l'utilisation pour désactiver les sites anioniques dans le substrat cellulosique et pour fournir un effet souhaité, dans lequel l'effet souhaité est choisi parmi un ou plusieurs d'un effet biocide, d'une meilleure sensation sur la peau, d'une peau plus saine et d'un assouplissement de tissus.

10. Procédé de désinfection d'une surface inanimée, le procédé comprenant l'humidification de la surface, puis son essuyage avec un applicateur selon l'une quelconque des revendications 1 à 8.

11. Procédé de désinfection d'une surface inanimée, le procédé comprenant l'humidification d'un applicateur selon l'une quelconque des revendications 1 à 8, puis l'essuyage de la surface avec l'applicateur humidifié, dans lequel l'applicateur comprend du papier ou du tissu, et selon lequel permettant l'application d'une espèce cationique bioactive sur la surface.

12. Procédé de désinfection de la peau, le procédé comprenant l'humidification de la peau puis son essuyage avec un applicateur selon l'une quelconque des revendications 1 à 8.

13. Procédé de désinfection de la peau, le procédé comprenant l'humidification d'un applicateur selon l'une quelconque des revendications 1 à 8, puis l'essuyage de la peau avec l'applicateur humidifié, dans lequel l'applicateur comprend du papier ou du tissu, et selon lequel permettant l'application d'une espèce cationique bioactive sur la peau.
